# EUROPÄISCHE PATENTSCHRIFT

(11) **EP 2 869 867 B1**
(45) Veröffentlichungstag und Bekanntmachung des Hinweises auf die Patenterteilung: **30.08.2017**
(21) Anmeldenummer: 13733956.0
(22) Anmeldetag: 03.07.2013
(51) Int. Cl.: A61M 1/36, G01M 3/16, G01M 3/18

(54) **VORRICHTUNG ZUR ERKENNUNG VON FEUCHTIGKEIT FÜR EINE VORRICHTUNG ZUR ÜBERWACHUNG EINES ZUGANGS ZU EINEM PATIENTEN**
HUMIDITY DETECTION DEVICE FOR A DEVICE FOR MONITORING ACCESS TO A PATIENT
DISPOSITIF POUR LA DÉTECTION DE L'HUMIDITÉ POUR UN DISPOSITIF DESTINÉ À LA SURVEILLANCE DE L'ACCÈS À UN PATIENT

(30) Priorität: 09.07.2012 DE 102012013473; 09.07.2012 US 201261669175 P
(43) Veröffentlichungstag der Anmeldung: 13.05.2015
(73) Patentinhaber: Fresenius Medical Care Deutschland GmbH, 61352 Bad Homburg (DE)
(72) Erfinder: SCHRÖRS, Alexander, 60389 Frankfurt (DE); HEPPE, John, 66606 St. Wendel (DE)
(74) Vertreter: Oppermann, Frank
(86) Internationale Anmeldenummer: PCT/EP2013/001947
(87) Internationale Veröffentlichungsnummer: WO 2014/008991

(56) Entgegenhaltungen:
- WO-A1-99/24145
- WO-A1-2011/116943
- WO-A2-2009/075592
- DE-B3-102006 011 313
- US-A1- 2002 198 483

## Beschreibung

Die Erfindung betrifft eine Vorrichtung zur Erkennung von Feuchtigkeit für eine Vorrichtung zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, insbesondere zur Überwachung des Gefäßzugangs bei einer extrakorporalen Blutbehandlung, insbesondere zur Überwachung eines zentralvenösen Katheters bei der akuten Dialyse. Darüber hinaus betrifft die Erfindung eine Vorrichtung zur Überwachung eines Zugangs zu einem Patienten, die über eine Vorrichtung zur Erkennung von Feuchtigkeit verfügt. Des Weiteren betrifft die Erfindung ein Verfahren zur Herstellung einer Vorrichtung zur Erkennung von Feuchtigkeit zum Anschluss an eine Vorrichtung zur Überwachung eines Patientenzugangs.

Auf dem Gebiet der Medizintechnik sind verschiedene Einrichtungen bekannt, mit denen über eine Schlauchleitung Patienten Flüssigkeiten entnommen oder Flüssigkeiten den Patienten zugeführt werden können. Dabei erfolgt der Zugang zu den Patienten im Allgemeinen mit einem Katheter zum Einführen in Körperorgane oder einer Kanüle zum Punktieren von Gefäßen. Während der Untersuchung oder Behandlung ist ein ordnungsgemäßer Zugang zu dem Patienten sicher zu stellen. Daher ist es erforderlich, den Patientenzugang zu überwachen.

Einen ordnungsgemäßen Zugang zu dem Patienten setzen insbesondere auch die extrakorporalen Blutbehandlungsvorrichtungen voraus, die über einen extrakorporalen Blutkreislauf verfügen. Zu den bekannten extrakorporalen Blutbehandlungsvorrichtungen zählen beispielsweise Dialysevorrichtungen und Zellseparatoren, die einen Zugang zu dem Gefäßsystem des Patienten erforderlich machen. Bei der extrakorporalen Blutbehandlung wird dem Patienten über eine arterielle Schlauchleitung mit einer arteriellen Punktionskanüle Blut entnommen, das dem Patienten über eine venöse Schlauchleitung mit einer venösen Punktionskanüle wieder zugeführt wird. Bei der akuten Dialyse auf Intensivstationen wird als Patientenzugang ein zentralvenöser Katheter am Hals des Patienten eingesetzt.

Trotz regelmäßiger Überwachung des Gefäßzugangs durch das Krankenhauspersonal besteht grundsätzlich die Gefahr, dass die Punktionskanüle unbemerkt aus dem Blutgefäß des Patienten herausrutscht oder sich eine Schlauchverbindung löst. Zur Überwachung des Gefäßzugangs sind verschiedene Vorrichtungen unterschiedlicher Ausbildung bekannt. Die bekannten Überwachungsvorrichtungen greifen im Allgemeinen auf die standardmäßig in den Blutbehandlungsvorrichtungen vorhandenen Sicherheitsvorrichtungen zurück, die bei einem nicht ordnungsgemäßen Gefäßzugang eine sofortige Unterbrechung des extrakorporalen Blutkreislaufs auslösen.

Zum Anschluss von Kathetern und Schlauchleitungen für die Herstellung eines Patientenzugangs findet im Allgemeinen das im medizinischen Bereich bekannte Luer-Verbindungssystem Verwendung, dessen Verbindungsteile einen Innen- und einen Außenkonus umfassen. Dieses Verbindungssystem wird als Luer-Lock-Verbindung bezeichnet, wenn der Innen- und Außenkonus zur Sicherung der Verbindung um ein Schraubgewinde erweitert ist. Obwohl die Luer-Lock-Verbindungen eine sehr hohe Sicherheit bieten, hat sich in der Praxis gezeigt, dass sich die Verbindungsteile bei unsachgemäßer Handhabung, bei Materialfehlern oder zu häufigem Gebrauch lösen oder Mikrorisse im Material auftreten können. Die US 2010/0228231 schlägt daher vor, die Verbindungsteile eines Luer-Lock-Verbindungssystems mit einer zusätzlichen Fixierung der Verbindungsteile gegen unbeabsichtigtes Lösen zu sichern.

Es sind Vorrichtungen zur Überwachung eines Gefäßzugangs bekannt, die über eine Vorrichtung zur Erkennung von Feuchtigkeit verfügen, um an der Punktionsstelle das Austreten von Blut erkennen zu können. Die bekannten Vorrichtungen zur Erkennung von Feuchtigkeit, die bei den bekannten Überwachungsvorrichtungen für den Patientenzugang Verwendung finden, sind als auf die Punktionsstelle aufzulegendes Pad ausgebildet. Das Pad besteht aus einem saugfähigen Material, in das ein Feuchtigkeitssensor eingebettet ist.

Vorrichtungen zum Detektieren von Feuchtigkeit aus einem saugfähigen Material, das auf die Haut des Patienten aufgelegt wird, sind beispielsweise aus der WO 2006/008866 A1, US 2005/0038325 A1 und US 6,445,304 B1 bekannt. Die bekannten Pads zeichnen sich dadurch aus, dass der Feuchtigkeitssensor in das saugfähige Material eingebettet ist und das saugfähige Material auf die Haut des Patienten aufgelegt wird.

Die WO 99/24145 beschreibt eine Vorrichtung zur Erkennung von Feuchtigkeit, die ein mit einem Deckel verschließbares Gehäuse aufweist, in dem ein Feuchtigkeitssensor angeordnet ist. Zur Durchführung der Kanülen und Schlauchleitungen sind in dem Gehäuse Durchbrüche vorgesehen. Nachteilig ist, dass das Gehäuse mit dem Feuchtigkeitssensor in großen Stückzahlen relativ kostspielig herzustellen ist und in der Praxis nur relativ schwierig zu handhaben ist.

Die US 2002/198483 A1 beschreibt eine Vorrichtung zur Erkennung von Feuchtigkeit, die einen an der Haut des Patienten zu befestigenden Feuchtigkeitssensor und einen Sensorhalter zur Befestigung des Sensors aufweist. Der Sensorhalter kann an der Punktionsstelle mit einem oder mehreren Bändern befestigt werden, die aus unterschiedlichen Materialien, beispielsweise starren oder elastischen Materialien, bestehen können.

Aus der WO 2009/075592 A2 ist eine Vorrichtung bekannt, die einen bandförmigen Feuchtigkeitssensor aufweist, der beispielsweise um den Arm des Patienten gelegt werden kann. Der bandförmige Feuchtigkeitssensor wird an dem Arm des Patienten mit einem in der Art einer Gürtelschnalle ausgebildeten Befestigungselement befestigt.

Der Erfindung liegt die Aufgabe zugrunde, eine in großen Stückzahlen kostengünstig herzustellende Vorrichtung zur Erkennung von Feuchtigkeit zu schaffen, die einfach zu handhaben ist und einen hohen Tragekomfort bietet. Eine weitere Aufgabe der Erfindung liegt darin, eine Vorrichtung zur Überwachung eines Zugangs zu einem Patienten mit einer derartigen Vorrichtung zur Erkennung von Feuchtigkeit zu schaffen. Eine Aufgabe der Erfindung ist auch ein Verfahren zur kostengünstigen Herstellung einer Vorrichtung zur Erkennung von Feuchtigkeit in großen Stückzahlen anzugeben.

Die Lösung dieser Aufgaben erfolgt erfindungsgemäß mit den Merkmalen der unabhängigen Ansprüche. Die Gegenstände der abhängigen Ansprüche betreffen vorteilhafte Ausführungsformen der Erfindung.

Die erfindungsgemäße Vorrichtung zur Erkennung von Feuchtigkeit weist einen Feuchtigkeitssensor auf, der als eine elektrisch leitende Struktur ausgebildet ist. Der Feuchtigkeitssensor der erfindungsgemäßen Vorrichtung wird an eine Vorrichtung zur Überwachung eines Zugangs zu einem Patienten angeschlossen.

Die erfindungsgemäße Vorrichtung zur Erkennung von Feuchtigkeit zeichnet sich dadurch aus, dass zumindest ein Teilstück oder Abschnitt der Vorrichtung als ein federndes Befestigungselement mit eine Schlauchleitung und/oder ein Verbindungssystem einer Schlauchleitung umgreifenden Abschnitten ausgebildet ist. Das in der Art eines Clips ausgebildete Befestigungselement erlaubt es, die Vorrichtung zur Erkennung von Feuchtigkeit an einer Schlauchleitung oder einem Schlauchleitungsverbindungssystem schnell und sicher zu befestigen. Zusätzliche Befestigungsmittel sind hierzu nicht erforderlich. Beispielsweise ist es nicht erforderlich, die Vorrichtung mit einem Klebeband an der Schlauchleitung, dem Verbindungssystem oder der Haut des Patienten zu fixieren. Dadurch wird die Handhabung der Vorrichtung vereinfacht.

Die erfindungsgemäße Vorrichtung kann insbesondere in dem Bereich der Schlauchleitung befestigt werden, an dem sich ein Verbindungssystem, beispielsweise ein Luer-Verbindungssystem befindet. Hierzu braucht die Vorrichtung nur um das Verbindungssystem gelegt zu werden, so dass sich die Verbindungsstelle auf Leckagen überwachen lässt.

Während eine zusätzliche Sicherung einer Luer-Verbindung, die in der US 2010/0228231 beschrieben ist, nur das Lösen der Verbindung verhindern kann, erlaubt die erfindungsgemäße Vorrichtung auch die Erkennung eines langsamen Blutverlustes, beispielsweise in Folge von Handhabungs- oder Materialfehlern der Schlauchverbindung.

Darüber hinaus ist es von Vorteil, dass sich die erfindungsgemäße Vorrichtung nach der Behandlung wieder leicht von der Schlauchleitung abnehmen lässt, was insbesondere bei der Verwendung zur Überwachung eines zentralvenösen Katheters von Bedeutung ist. Die Vorrichtung kann auch während der Behandlung leicht von der Schlauchleitung gelöst werden. Auch kann die erfindungsgemäße Vorrichtung während der Behandlung leicht gegen eine neue Vorrichtung ausgetauscht werden.

Die erfindungsgemäße Vorrichtung kann über einen oder mehrere federnde Befestigungselemente verfügen. Mit mehreren federnden Befestigungselementen kann die Sicherheit der Fixierung der Vorrichtung an der Schlauchleitung noch erhöht werden.

Die erfindungsgemäße Vorrichtung kann unterschiedliche Abmessungen haben, um an die unterschiedlichen Dimensionen der Gefäßzugänge angepasst zu sein. Entscheidend ist, dass die Abschnitte des federnden Befestigungselements die Schlauchleitung oder das Verbindungssystem mit dem jeweiligen Durchmesser sicher umgreifen.

Die erfindungsgemäße Vorrichtung ist als ein einstückiger, zumindest bereichsweise, federelastischer Körper ausgebildet. Vorzugsweise ist die Vorrichtung als ein langgestreckter Körper ausgebildet, der sich einschnappend an der Schlauchleitung oder des Schlauchleitungsverbindungssystems fixieren lässt. Eine weitere bevorzugte Ausführungsform sieht vor, dass das federnde Befestigungselement als ein ringförmiger Körper ausgebildet ist, der in Längsrichtung geschlitzt ist. Zum Einlegen der Schlauchleitung braucht das Befestigungselement nur aufgespreizt zu werden, wobei sich das Befestigungselement wieder unter der Federspannung schließt. Dabei ist vorteilhaft, dass bei einer Zugbeanspruchung der elektrischen Verbindungsleitung zwischen der Vorrichtung zur Erkennung von Feuchtigkeit und der Überwachungsvorrichtung sich die erfindungsgemäße Vorrichtung lösen kann, so dass der Patientenzugang nicht auf Zug beansprucht wird.

Die Handhabung der erfindungsgemäßen Vorrichtung wird noch dadurch verbessert, dass der ringförmige Körper im Bereich des Schlitzes Abschnitte aufweist, die zu beiden Seiten nach außen umgebogen sind. Die umgebogenen Abschnitte bilden eine Führung zum Einlegen der Schlauchleitung.

Die erfindungsgemäße Vorrichtung weist vorzugsweise eine dem Patienten zugewandte außenliegende für Flüssigkeit nicht durchlässige Schicht und eine dem Patienten abgewandte innenliegende für Flüssigkeit saugfähige Schicht auf. Dadurch wird erreicht, dass an der Schlauchverbindungsstelle austretendes Blut unmittelbar an den Feuchtigkeitssensor gelangt. Weiterhin wird vermieden, dass Schweiß von der Haut des Patienten zu dem Feuchtigkeitssensor gelangt, so dass Fehlalarme vermieden werden.

Eine besonders bevorzugte Ausführungsform der Erfindung sieht vor, dass das saugfähige Material ein textiles Material ist, das die elektrisch leitende Struktur aufweist. Die elektrisch leitende Struktur kann in das textile Material eingebettet oder auf das textile Material aufgebracht sein.

Die besonderen Vorteile der erfindungsgemäßen Vorrichtung liegen insbesondere darin, dass sich die Vorrichtung aus einem Verbund von einem vorzugsweisen textilen Material mit einer elektrisch leitfähigen Struktur und einem thermisch verformbaren Trägermaterial in großen Stückzahlen einfach dadurch herstellen lässt, dass der Verbund aus dem textilen Material und dem Trägermaterial unter Ausbildung der federnden Befestigungselemente thermisch verformt wird. Mit diesem Herstellungsverfahren lassen sich die erfindungsgemäßen Vorrichtungen in großen Stückzahlen kostengünstig herstellen.

Die elektrisch leitende Struktur des Feuchtigkeitssensors weist vorzugsweise mindestens eine elektrische Leiterbahn auf, die in das textile Material eingebettet ist. Vorzugsweise ist das textile Material ein Gewebe mit nicht-leitfähigen Kettfäden und nicht-leitfähigen Schussfäden sowie leitfähigen Kettfäden und leitfähigen Schussfäden, die derart angeordnet sind, dass die mindestens eine elektrische Leiterbahn ausgebildet ist. Ein derartiges Gewebe mit einer elektrisch leitfähigen Struktur ist in der WO 2011/116943 im Einzelnen beschrieben.

Die erfindungsgemäße Vorrichtung verfügt vorzugsweise über Anschlusskontakte zur elektrischen Kontaktierung des Feuchtigkeitssensors. Es ist aber auch möglich, dass eine elektrische Verbindungsleitung aus der erfindungsgemäßen Vorrichtung herausgeführt wird, die an die Überwachungsvorrichtung angeschlossen wird.

Eine weitere bevorzugte Ausführungsform sieht vor, dass die Anschlusskontakte am Endstück eines langgestreckten Teilstücks der Vorrichtung ausgebildet sind. Dadurch sind die Anschlusskontakte von dem Feuchtigkeitssensor ausreichend räumlich getrennt. Daher braucht der Bereich der elektrischen Kontaktierung im Gegensatz zu dem Bereich, an dem sich der Feuchtigkeitssensor befindet, nicht steril zu sein. Dies ist insbesondere bei der Überwachung von zentralvenösen Kathetern von Vorteil.

Bei einer besonders bevorzugten Ausführungsform weist die erfindungsgemäße Vorrichtung zwei im Abstand zueinander angeordnete Teilstücke auf, die als federnde Befestigungselemente ausgebildet sind. Diese Ausführungsform erlaubt die Fixierung der erfindungsgemäßen Vorrichtung mit den Verbindungsteilen einer Schlauchleitung, die insbesondere Luer-Lock-Kollektoren sind. Der Abstand der beiden federnden Befestigungselemente ist derart bemessen, dass die Verbindungsteile der Schlauchleitung zwischen den Befestigungselementen zu liegen kommen.

Eine weitere bevorzugte Ausführungsform sieht ein drittes federndes Befestigungselement vor, das zwischen dem ersten und zweiten Befestigungselement angeordnet ist. Das dritte federnde Befestigungselement hat vorzugsweise eine größere Länge als das erste und zweite Befestigungselement. An dem dritten federnden Befestigungselement kann die Vorrichtung an den Verbindungsteilen der Schlauchleitung fixiert werden, in dem die Verbindungsteile von dem dritten Befestigungselement einschnappend umschlossen werden. Dadurch wird die Sicherheit der Fixierung der erfindungsgemäßen Vorrichtung an der Schlauchleitung noch weiter erhöht.

Die erfindungsgemäße Vorrichtung aus dem Verbund von einem Textilmaterial und einem verformbaren Kunststoffmaterial kann ohne großen technischen Aufwand sterilisiert werden und als Einwegsensor in einer geeigneten Verpackung steril bereitgestellt werden.

Die erfindungsgemäße Vorrichtung zur Überwachung eines Zugangs zu einem Patienten, insbesondere zur Überwachung eines zentralvenösen Katheters verfügt über die erfindungsgemäße Vorrichtung zur Erkennung von Feuchtigkeit, die an die Überwachungsvorrichtung angeschlossen wird. Die Überwachungsvorrichtung kann einen akustischen und/oder optischen und/oder taktilen Alarm auslösen, wenn Feuchtigkeit detektiert wird. Auch kann ein Steuersignal für einen Eingriff in die Steuerung der Einrichtung erzeugt werden, mit der über eine Schlauchleitung eine Flüssigkeit dem Patienten zugeführt und/oder eine Flüssigkeit von dem Patienten abgeführt wird.

Die erfindungsgemäße Vorrichtung zur Überwachung eines Patientenzugangs kann eine separate Einheit bilden oder Bestandteil der Einrichtung sein, mit der dem Patienten eine Flüssigkeit zugeführt und/oder von dem Patienten Flüssigkeit abgeführt wird, insbesondere Bestandteil der extrakorporalen Blutbehandlungsvorrichtung sein. Wenn die erfindungsgemäße Überwachungsvorrichtung Bestandteil der Blutbehandlungsvorrichtung ist, kann die Überwachungsvorrichtung von bestimmten Baugruppen oder Bauteilen Gebrauch machen, die in der Blutbehandlungsvorrichtung ohnehin vorhanden sind.

Im Folgenden wird ein Ausführungsbeispiel der erfindungsgemäßen Vorrichtung unter Bezugnahme auf die Zeichnungen näher erläutert.

Es zeigen:
- Fig. 1: die wesentlichen Komponenten einer Blutbehandlungsvorrichtung, die über eine Vorrichtung zur Überwachung eines Gefäßzugangs verfügt,
- Fig. 2: die erfindungsgemäße Vorrichtung zur Erkennung von Feuchtigkeit in perspektivischer Darstellung,
- Fig. 3: den Ausschnitt A von Fig. 2 in vergrößerter Darstellung,
- Fig. 4: einen Schnitt durch den Verbund von textilem Material und thermisch verformbaren Trägermaterial,
- Fig. 5: ein erstes Ausführungsbeispiel der erfindungsgemäßen Vorrichtung vor der Verformung des Verbundes von textilem Material und thermisch verformbarem Trägermaterial,
- Fig. 6: ein zweites Ausführungsbeispiel der erfindungsgemäßen Vorrichtung vor der Verformung des Materialverbundes und
- Fig. 7: eine schematische Darstellung zur Veranschaulichung der Prozessschritte zur Herstellung der erfindungsgemäßen Vorrichtung.

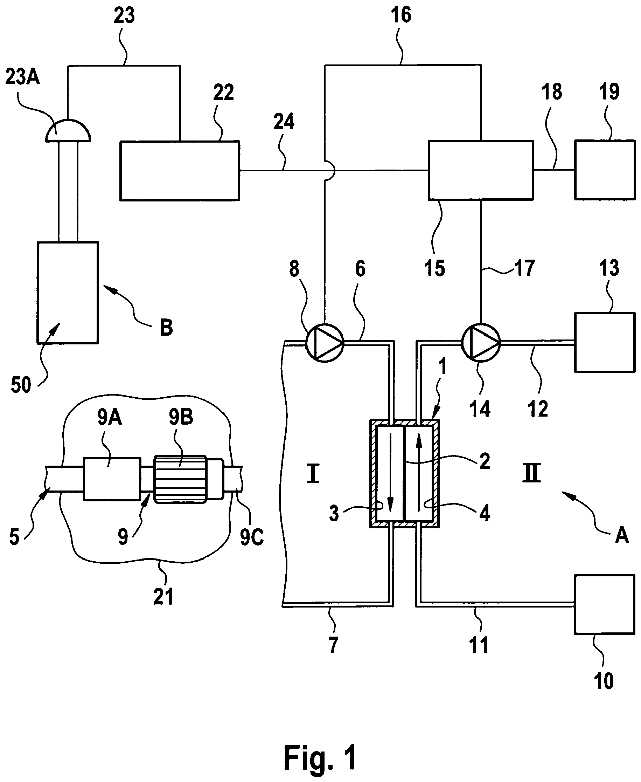

Fig. 1 zeigt die wesentlichen Komponenten einer Blutbehandlungsvorrichtung, insbesondere Hämodialysevorrichtung A für die Akutdialyse, die über eine Vorrichtung B zur Überwachung eines Gefäßzugangs, insbesondere eines Gefäßzugangs mit einem zentralvenösen Katheter verfügt. Bei dem vorliegenden Ausführungsbeispiel ist die Überwachungsvorrichtung B Bestandteil der Hämodialysevorrichtung A. Zunächst wird die Dialysevorrichtung unter Bezugnahme auf Fig. 1 beschrieben.

Die Hämodialysevorrichtung A weist einen Dialysator 1 auf, der durch eine semipermeable Membran 2 in eine Blutkammer 3 und eine Dialysierflüssigkeitskammer 4 unterteilt ist. Der Gefäßzugang zum Patienten erfolgt mit einem zentralvenösen Katheter 5, der am Hals des Patienten angeschlossen wird. Der zentralvenöse Katheter 5 ist Teil des nur andeutungsweise dargestellten extrakorporalen Blutkreislaufs I, der die Blutkammer 3 des Dialysators 1 einschließt und Schlauchleitungen 6, 7 umfasst. Zum Fördern des Bluts im extrakorporalen Kreislauf ist ein Blutpumpe 8 vorgesehen.

Der Dialysierflüssigkeitskreislauf II der Dialysevorrichtung A umfasst eine Dialyseflüssigkeitsquelle 10, an der eine Dialysierflüssigkeitszuführleitung 11 angeschlossen ist, die zu dem Einlass der Dialysierflüssigkeitskammer 4 des Dialysators 1 führt. Von dem Auslass der Dialysierflüssigkeitskammer 4 des Dialysators 1 geht eine Dialysierflüssigkeitsabführleitung 12 ab, die zu einem Auslass 13 führt. In die Dialysierflüssigkeitsabführleitung 12 ist eine Dialysierflüssigkeitspumpe 14 geschaltet.

Die Steuerung der Dialysevorrichtung übernimmt eine zentrale Steuereinheit 15, die über Steuerleitungen 16, 17 die Blut- und Dialysierflüssigkeitspumpe 8, 14 ansteuert. Die zentrale Steuereinheit 15 ist über eine Datenleitung 18 mit einer Alarmeinheit 19 verbunden, die bei einem Störfall einen optischen und/oder akustischen und/oder taktilen Alarm gibt.

Die nur schematisch dargestellte Überwachungsvorrichtung B dient bei dem vorliegenden Ausführungsbeispiel zur Überwachung einer Luer-Lock-Verbindung 9 mit den Verbindungsteilen 9A und 9B zum Anschluss des zentralvenösen Katheters 5 an eine Schlauchleitung 9C des extrakorporalen Blutkreislaufs I. Die Überwachungsvorrichtung B verfügt über eine Vorrichtung 50 zur Erkennung von Feuchtigkeit, die an der Schlauchverbindungsstelle 21 angeordnet wird. Dieser Feuchtigkeitssensor 50 ist in Fig. 1 nur schematisch dargestellt. Darüber hinaus verfügt die Überwachungsvorrichtung B über eine Auswerteinrichtung 22, die über eine Verbindungsleitung 23 mit dem Feuchtigkeitssensor 50 elektrisch verbunden ist. Die Verbindungsleitung 23 ist mit einem elektrischen Verbindungsstück 23A an den Feuchtigkeitssensor 50 angeschlossen.

Über eine Datenleitung 24 ist die Auswerteinrichtung 22 mit der zentralen Steuereinheit 15 der Dialysevorrichtung A verbunden. Für den Fall, dass Blut aus der Schlauchverbindungsstelle 21 austritt und den Feuchtigkeitssensor 50 befeuchtet, erzeugt die Auswerteinrichtung 22 der Überwachungsvorrichtung B ein Steuersignal, das die zentrale Steuereinheit 15 über die Datenleitung 24 empfängt, die einen Eingriff in die Blutbehandlung vornimmt. Die Steuereinheit 15 stoppt die Blutpumpe 8 und erzeugt ein Alarmsignal, sodass die Alarmeinheit 19 einen akustischen und/oder optischen und/oder taktilen Alarm gibt.

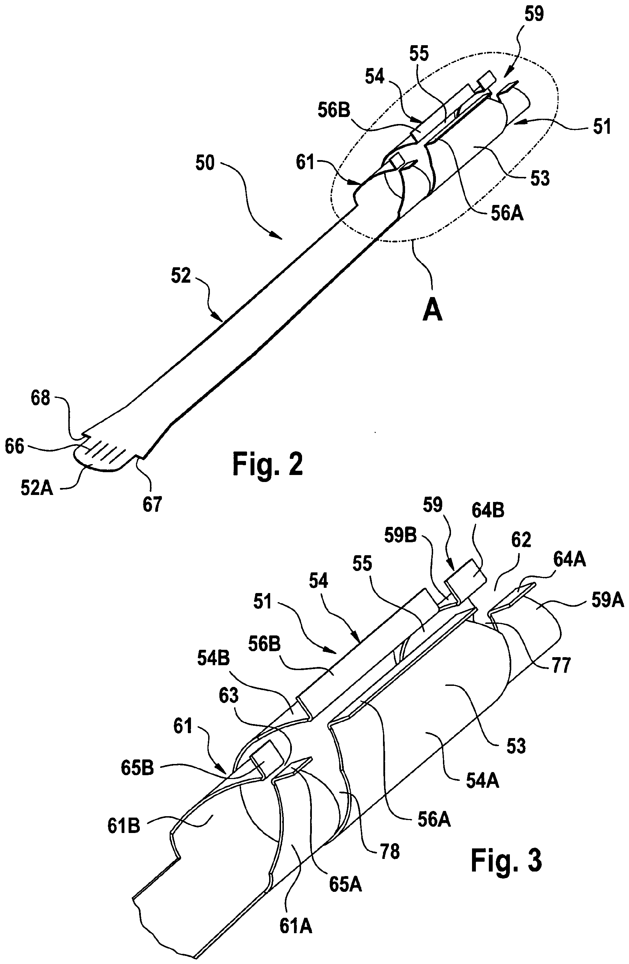

Nachfolgend wird ein Ausführungsbeispiel der erfindungsgemäßen Vorrichtung zur Erkennung von Feuchtigkeit unter Bezugnahme auf die Figuren 2 bis 6 im Einzelnen beschrieben.

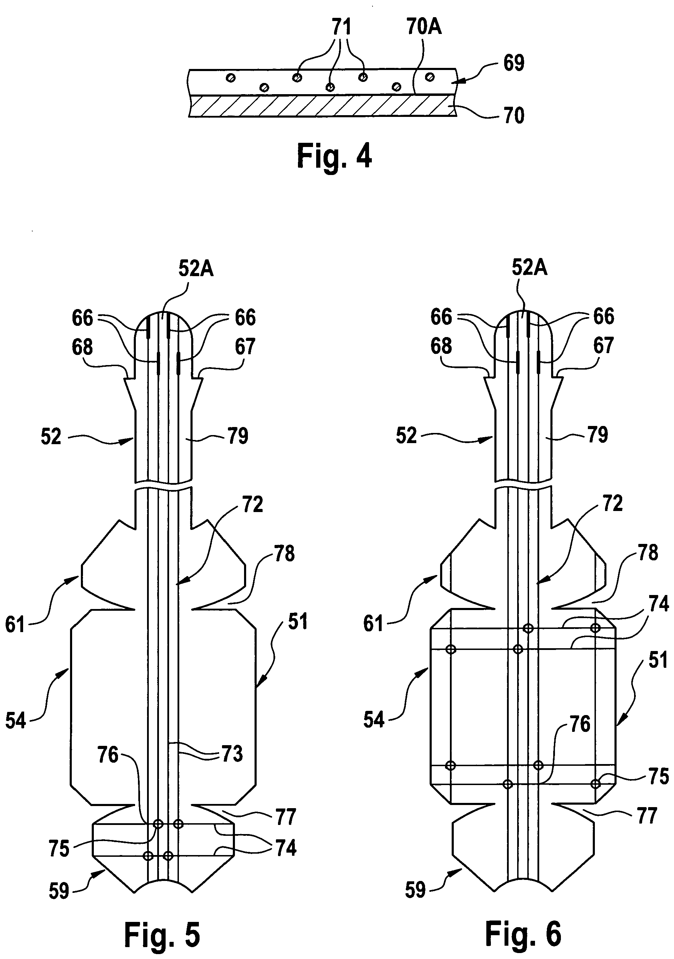

Fig. 2 zeigt ein Ausführungsbeispiel der Vorrichtung zur Erkennung von Feuchtigkeit 50 in perspektivischer Darstellung. Die Vorrichtung 50 ist durch thermisches Verformen eines Verbundes von einem saugfähigen textilen Material und einem thermisch verformbaren Trägermaterial hergestellt. Der Materialverbund und das Herstellungsverfahren werden unter Bezugnahme auf die Figuren 4 bis 7 im Einzelnen beschrieben.

Die Vorrichtung 50 zur Erkennung von Feuchtigkeit ist als ein langgestreckter Körper ausgebildet, der sich an einer nicht dargestellten Schlauchleitung oder einem Schlauchleitungsverbindungssystem einschnappend fixieren lässt. Die Vorrichtung gliedert sich in einen proximalen Abschnitt 51 zur Fixierung einer nicht dargestellten Schlauchleitung oder einem Verbindungssystem der Schlauchleitung und einen distalen Abschnitt 52, an den ein Anschlussstück für den elektrischen Anschluss an die Überwachungsvorrichtung angeschlossen wird.

Der mittlere Abschnitt 53 des proximalen Abschnitts 51 ist als ein federndes Befestigungselement 54 für die nicht dargestellte Schlauchleitung oder das Schlauchleitungsverbindungssystem ausgebildet. Das mittlere Befestigungselement 54 ist ein hülsenförmiger Körper, der in Längsrichtung geschlitzt ist. Im Bereich des Schlitzes 55 weist das mittlere Befestigungselement 54 nach außen umgebogene Abschnitte 56A, 56B auf.

Fig. 3 zeigt den proximalen Abschnitt 51 der Vorrichtung zur Erkennung von Feuchtigkeit in vergrößerter Darstellung, in den die nicht dargestellte Schlauchleitung, die aus zwei Schlauchleitungsabschnitten besteht, die mittels Verbindungsteilen miteinander verbunden sind. Bei den Verbindungsteilen handelt es sich um die allgemein bekannten Luer-Verbindungsteile 9A und 9B (Fig. 1).

Die Luer-Lock-Verbindungsteile der Schlauchleitung werden von den beiden federnden Abschnitten 54A, 54B des mittleren Befestigungselements 54 umschlossen, so dass die Schlauchleitung fixiert ist. Der Innendurchmesser des mittleren Befestigungselements 54 entspricht daher etwa dem Außendurchmesser der Luer-Lock-Verbindungsteile. Das Einführen der Verbindungsteile wird durch die nach außen umgebogenen Abschnitte 56A, 56B des mittleren Befestigungselements 54 erleichtert, indem die beiden Abschnitte 54A, 54B des mittleren Befestigungselements 54 mit den sich an den Verbindungsteilen abstützenden Schrägflächen aufspreizen können. Die Länge des mittleren Befestigungselements 54 entspricht etwa der Länge beiden Verbindungsteile.

An das mittlere Befestigungselement 54 schließt sich ein weiteres proximales federndes Befestigungselement 59 und ein distales federndes Befestigungselement 61 an. Das proximale und distale Befestigungselement 59, 61 sind in Längsrichtung geschlitzte hülsenförmige Körper, die einen kleineren Innendurchmesser als das mittlere federnde Befestigungselement 54 haben. Das distale und proximale Befestigungselement haben den gleichen Innendurchmesser. Beide Befestigungselemente 59, 61 weisen die Schlauchleitung umgreifende Abschnitte 59A, 59B bzw. 61A, 61B auf. Im Bereich der Schlitze 62 bzw. 63 weisen beide Befestigungselemente 59, 61 Abschnitte 64A, 64B bzw. 65A, 65B auf, die nach außen umgebogen sind.

Das proximale und distale Befestigungselement 59, 61 ist von dem mittleren Befestigungselement 54 durch V-förmige Einschnitte 77, 78 getrennt. Da der Innendurchmesser des proximalen und distalen Befestigungselements 59, 61 dem Außendurchmesser der Schlauchleitung entspricht, ist die Schlauchleitung zusätzlich mit dem proximalen und distalen Befestigungselement fixiert. Gleichsam stellen das proximale und distale Befestigungselement Anschläge für die Verbindungsteile der Schlauchleitung dar, weil der Innendurchmesser des proximalen und distalen Befestigungselements 59, 61 kleiner als der Außendurchmesser der Verbindungsteile der Schlauleitung ist.

Wenn die Vorrichtung zur Erkennung von Feuchtigkeit auf Zug beansprucht werden sollte, kann sie mit einem durch den Abstand des proximalen und distalen Befestigungselements vorgegebenen Spiel leicht in Längsrichtung verschoben werden, so dass die Zugkraft kaum auf den zentralvenösen Katheter übertragen wird. Dies erfüllt die Funktion einer "Sollbruchstelle".

Der distale Abschnitt 52 der Vorrichtung zur Erkennung von Feuchtigkeit ist nicht thermisch verformt. Der distale Abschnitt 52 ist ein flacher Streifen 79, an dessen Endstück 52A das nicht dargestellte Anschlussstück der zu der Überwachungsvorrichtung führenden Verbindungsleitung angeschlossen wird. Auf der Oberseite des Endstücks 52A befinden sich die Anschlusskontakte 66 des Feuchtigkeitssensors der Vorrichtung zur Erkennung von Feuchtigkeit. An dem Endstück 52A sind seitliche Ansätze 67, 68 ausgebildet, die einen Anschlag für das nicht dargestellte Anschlussstück bilden. Der Feuchtigkeitssensor wird nachfolgend unter Bezugnahme auf die Figuren 4 bis 6 beschrieben.

Die erfindungsgemäße Vorrichtung zur Erkennung von Feuchtigkeit wird aus einem Materialverbund von einem saugfähigen textilen Material, insbesondere einem mehrlagigen Gewebe 69 und einem thermisch verformbaren Trägermaterial 70 hergestellt. Fig. 4 zeigt einen Schnitt durch den Materialverbund. Der Verbund zwischen Gewebe 69 und Trägermaterial 70 kann beispielsweise mittels einer Klebeschicht 70A erfolgen. Ein Laminieren unter hohem Druck kann dadurch umgangen werden. Eine zu starke Pressung beim Laminieren unter Druck könnte sich nämlich nachteilig auf die Leiterbahnstruktur in dem textilen Material auswirken bzw. die Leiterbahnen beschädigen.

Das Mehrlagengewebe besteht aus elektrisch leitfähigen und elektrisch nicht leitfähigen Kett- und Schussfäden (Monofilamente, Karbonfasern, versilbertes Polyamidgarn) die in Fig. 4 nur andeutungsweise dargestellt sind. Die elektrisch leitfähigen und elektrisch nicht-leitfähigen Kett- und Schussfäden 71 sind derart angeordnet, dass das Gewebe eine untere Lage, eine mittlere Lage und eine obere Lage aufweist.

Die elektrisch leitende Struktur von Leiterbahnen bildet den Feuchtigkeitssensor der Vorrichtung zur Erkennung von Feuchtigkeit. Ein derartiger aus Kett- und Schussfäden bestehender Feuchtigkeitssensor ist in der WO 2011/116943 im Einzelnen beschrieben, auf die ausdrücklich Bezug genommen wird.

Die Figuren 5 und 6 zeigen in der Draufsicht den Materialverbund der Vorrichtung zur Erkennung von Feuchtigkeit vor der thermischen Verformung. Die beiden Ausführungsbeispiele von Fig. 5 und Fig. 6 unterscheiden sich nur durch das Layout des Feuchtigkeitssensors. Für die Bezeichnung der einzelnen Abschnitte der Vorrichtung zur Erkennung von Feuchtigkeit werden daher die Bezugszeichen der Figuren 1 und 2 verwendet.

Die elektrisch leitende Struktur 72 des Feuchtigkeitssensors besteht aus längslaufenden Leiterbahnen 73 und querverlaufenden Leiterbahnen 74, die an den mit 75 bezeichneten Kreuzungspunkten elektrisch kontaktiert und an den mit 76 bezeichneten Kreuzungspunkten elektrisch voneinander isoliert sind. An den Enden der längslaufenden Leiterbahnen 73 befinden sich die bei diesem Ausführungsbeispiel versetzt zueinander angeordneten Anschlusskontakte 66.

Die in Fig. 6 gezeigte Ausführungsform des Feuchtigkeitssensors hat eine höhere Sensitivität als die Ausführungsform von Fig. 5, da die Anzahl der Leiterbahnen größer ist. Darüber hinaus ist der Feuchtigkeitssensor von Fig. 6 auch in den Randbereichen des mittleren Befestigungselements 54 sensitiv. Die Sensitivität des Feuchtigkeitssensors von Fig. 5 ist allerdings ausreichend, da das Gewebe ein für Flüssigkeit, beispielsweise Blut, saugfähiges Material ist.

Die V-förmigen Einschnitte 77, 78 zwischen den federnden Befestigungselementen erleichtern das Einführen der Schlauchleitung. Da die Vorrichtung zur Erkennung von Feuchtigkeit einschnappend an der Schlauchleitung festgelegt ist, kann die Vorrichtung leicht wieder von der Schlauchleitung abgezogen werden. Der jeweilige Durchmesser des proximalen Befestigungselements 59 und des distalen Befestigungselements 61 und des mittleren Befestigungselements 54 können unterschiedlich sein. Insbesondere können die Durchmesser des proximalen und des distalen Befestigungselements 59, 61 kleiner sein als der Durchmesser des mittleren Befestigungselements 54. Die V-förmigen Einschnitte 77, 78 in den Figuren 5 und 6 weisen in der dargestellten Abwicklung Radien auf, die jeweils entsprechend dem Durchmesser des proximalen und distalen Befestigungselements 59, 61 angepasst werden können. Die Länge des streifenförmigen Abschnitts 52 ist derart bemessen, dass das Anschlussstück des Feuchtigkeitssensors ausreichend weit von dem sterilen Bereich entfernt ist.

Das thermisch verformbare Trägermaterial 70 ist ein Material, das unter Temperatureinwirkung eine bleibende Verformung behält und nach der Verformung federelastisch ist. Das Gewebe 69 kann beispielsweise mit einer steifen thermoplastischen Folie als Trägermaterial kaschiert werden. Die für die thermische Verformung erforderliche Temperatur ist abhängig von dem Trägermaterial. Die Temperatur sollte in einem Bereich liegen, der die Eigenschaften des Gewebes, beispielsweise die hydrophilen Eigenschaften, nicht beeinträchtigt und nicht zu einer Beschädigung der Kontakt- und Isolationsstellen etc. führt.

Es ist grundsätzlich auch möglich, mit einer entsprechenden Wärmebehandlung nur das textile Material plastisch zu verformen, um eine dreidimensionale Struktur zu erhalten.

Durch Einweben stärkerer Fäden in das Gewebe kann die mechanische Stabilität dabei verbessert werden. Allerdings hat der Materialverbund aus einer thermoplastischen Folie 70 und einem Gewebe 69 im Hinblick auf die mechanischen Eigenschaften wie Elastizität und Steifigkeit, die für die Handhabung und Beständigkeit des Sensors von Bedeutung sind, entscheidende Vorteile. Außerdem erfüllt die thermoplastische Folie die Funktion der Feuchtigkeitsbarriere gegen Feuchtigkeit von außen.

Das Trägermaterial 70 aus Kunststoff kann beispielsweise eine Kunststofffolie mit einer Dicke von ca. 250µm sein, die aus zwei Polyesterfolien mit einer Dicke von ca. 125µm hergestellt werden kann, indem die Folien mit ihrer Siegelschicht einander zugewandt miteinander laminiert werden. In einem weiteren Kaschierprozess wird das Trägermaterial 70 mit dem Gewebe 69 verbunden. Nach Ausschneiden der in den Figuren 5 und 6 gezeigten Kontur wird der Materialverbund derart thermisch verformt, so dass die in den Figuren Fig. 2 und 3 gezeigte dreidimensionale Form entsteht. Die thermische Verformung kann beispielsweise unter der Einwirkung von Heißluft oder Infrarotstrahlung erfolgen, wodurch die Siegelschicht zwischen den Polyesterfolien aufschmilzt. Die dreidimensionale Form kann beispielsweise durch einen geeigneten Kern vorgegeben werden, der bei dem vorliegenden Ausführungsbeispiel eine zylindrische Form hat. Nach dem Aufschmelzen der Siegelschicht der Polyesterfolien nimmt der Materialverbund dann die in den Figuren 2 und 3 gezeigte Form an. Es ist aber auch möglich, eine thermoplastische Folie zu verformen.

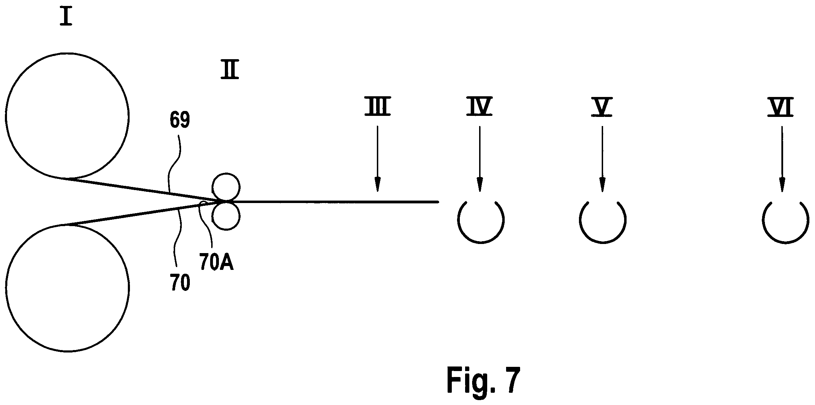

Fig. 7 zeigt die einzelnen Prozessschritte zur Herstellung der erfindungsgemäßen Vorrichtung zur Erkennung von Feuchtigkeit.

In einem ersten Produktionsschritt I werden das Gewebe 69 und das Trägermaterial 70 in Bahnen als Rollenware hergestellt. In einem nachfolgenden Kaschierprozess II werden Gewebe 69 und Trägermaterial 70 durch eine Klebeschicht 70A miteinander verbunden. Daraufhin werden in einem weiteren Produktionsschritt III die in den Figuren 5 oder 6 gezeigten Ausschnitte aus der Materialbahn vereinzelt. Daraufhin folgt der Prozessschritt der thermischen Verformung (IV) der vereinzelten Ausschnitte. Während der Verformung wird die ausgeschnittene Abwicklung durch ein Werkzeug in der dreidimensionalen Form fixiert. Die Form der Werkzeuge für Matrize und Kern bestimmen dabei die Außenkontur des Sensors. Matrize und Kern werden während des thermischen Verformens beheizt und danach gekühlt. Daran schließt sich der Prozessschritt V der Inprozesskontrolle und Freigabe sowie der Prozessschritt VI der Konfektionierung an.

Die einzelnen Vorrichtungen zur Erkennung von Feuchtigkeit können in einer formfesten Verpackung vor Beschädigungen geschützt werden. Die Vorrichtungen können aber auch in flexiblen Verpackungen konfektioniert werden, wenn Einlegeteile zum Schutz vor dem Zusammendrücken verwendet werden.

## Patentansprüche

1. Vorrichtung zur Erkennung von Feuchtigkeit für eine Vorrichtung zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, wobei die Vorrichtung zur Erkennung von Feuchtigkeit einen Feuchtigkeitssensor aufweist, der als eine elektrisch leitende Struktur (72) ausgebildet ist,
**dadurch gekennzeichnet,**
**dass** die Vorrichtung zur Erkennung von Feuchtigkeit als ein einstückiger zumindest bereichsweise federelastischer Körper ausgebildet ist und dass zumindest ein Teilstück oder Abschnitt der Vorrichtung zur Erkennung von Feuchtigkeit als ein federndes Befestigungselement (54) mit eine Schlauchleitung oder ein Verbindungssystem der Schlauchleitung umgreifenden Abschnitten (54A, 54B) ausgebildet ist.

2. Vorrichtung nach Anspruch 1, **dadurch gekennzeichnet, dass** die Vorrichtung zur Erkennung von Feuchtigkeit als ein langgestreckter Körper ausgebildet ist

3. Vorrichtung nach Anspruch 1 oder 2, **dadurch gekennzeichnet, dass** das mindestens eine federnde Befestigungselement (54) als ein ringförmiger Körper ausgebildet ist, der in Längsrichtung geschlitzt ist.

4. Vorrichtung nach Anspruch 3, **dadurch gekennzeichnet, dass** der ringförmiger Körper im Bereich des Schlitzes (55) Abschnitte (56A, 56B) aufweist, die zu beiden Seiten nach außen umgebogen sind.

5. Vorrichtung nach einem der Ansprüche 1 bis 4, **dadurch gekennzeichnet**, die Vorrichtung zur Erkennung von Feuchtigkeit eine dem Patienten zugewandte außenliegende für Feuchtigkeit nicht durchlässige Schicht (70) und eine dem Patienten abgewandte innenliegende für Flüssigkeit saugfähige Schicht (69) aufweist.

6. Vorrichtung nach Anspruch 5, **dadurch gekennzeichnet, dass** die innenliegende für Flüssigkeit saugfähige Schicht (69) ein textiles Material ist, das die elektrisch leitende Struktur (72) aufweist.

7. Vorrichtung nach Anspruch 5 oder 6, **dadurch gekennzeichnet, dass** die außenliegende Schicht (70) eine Schicht aus einem federelastischen Kunststoff ist, der thermisch verformt ist.

8. Vorrichtung nach Anspruch 6 oder 7, **dadurch gekennzeichnet, dass** die elektrisch leitende Struktur (72) mindestens eine elektrische Leiterbahn (73, 74) aufweist, die in das textile Material (69) eingebettet ist.

9. Vorrichtung nach Anspruch 8, **dadurch gekennzeichnet, dass** das textile Material ein Gewebe (69) mit nicht-leitfähigen Kettfäden und nicht-leitfähigen Schussfäden (71) sowie leitfähigen Kettfäden und leitfähigen Schussfäden (71) ist, die derart angeordnet sind, dass die mindestens eine elektrische Leiterbahn (73, 74) ausgebildet ist.

10. Vorrichtung nach einem der Ansprüche 1 bis 9, **dadurch gekennzeichnet, dass** die Vorrichtung zur Erkennung von Feuchtigkeit Anschlusskontakte (66) zur elektrischen Kontaktierung des Feuchtigkeitssensors aufweist.

11. Vorrichtung nach Anspruch 10, **dadurch gekennzeichnet, dass** die Anschlusskontakte (66) am Endstück (52A) eines langgestreckten Teilstücks (79) der Vorrichtung zur Erkennung von Feuchtigkeit ausgebildet sind.

12. Vorrichtung nach einem der Ansprüche 1 bis 11, **dadurch gekennzeichnet**, die Vorrichtung zur Erkennung von Feuchtigkeit zwei im Abstand zueinander angeordnete Teilstücke oder Abschnitte aufweist, die als ein erstes und zweites federndes Befestigungselement (59, 61) ausgebildet sind.

13. Vorrichtung nach Anspruch 12, **dadurch gekennzeichnet, dass** die Vorrichtung zur Erkennung von Feuchtigkeit ein drittes federndes Befestigungselement (54) aufweist, das zwischen dem ersten und zweiten federnden Befestigungselement (59, 61) angeordnet ist.

14. Vorrichtung nach Anspruch 13, **dadurch gekennzeichnet, dass** das dritte federnde Befestigungselement (54) in Längsrichtung eine größere Länge als das erste und zweite federnde Befestigungselement (59, 61) hat.

15. Vorrichtung zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, **dadurch gekennzeichnet, dass** die Überwachungsvorrichtung (B) eine Vorrichtung (50) zur Erkennung von Feuchtigkeit nach einem der Ansprüche 1 bis 14 aufweist.

16. Verfahren zur Herstellung einer Vorrichtung zur Erkennung von Feuchtigkeit nach einem der Ansprüche 1 bis 14 mit folgenden Verfahrens schritten:
Aufbringen eines textilen Materials (69) mit einer elektrisch leitfähigen Struktur auf ein thermisch verformbares Trägermaterial (70),
Ausschneiden eines Verbundes mit einer vorgegebenen Kontur aus dem textilen Material und dem Trägermaterial,
Thermisches Verformen des ausgeschnittenen Verbundes aus dem textilen Material und dem Trägermaterial, und
Abkühlen des thermisch verformten Verbundes aus dem textilen Material und dem Trägermaterial.

## Claims

1. Device for detecting moisture for an arrangement for monitoring an access to a patient for a system by which, via a flexible line, a liquid is fed to a patient and/or a liquid is fed out from the patient, the device for detecting moisture having a moisture sensor which takes the form of an electrically conductive structure (72),
**characterised in that** the device for detecting moisture takes the form of a one-piece body which is resilient at least in a region or regions, and that at least a part or portion of the device for detecting moisture takes the form of a resilient attaching element (54) having parts (54A, 54B) which fit round a flexible line and/or a system for connecting the flexible line.

2. Device according to claim 1, **characterised in that** the device for detecting moisture takes the form of an elongated body.

3. Device according to claim 1 or 2, **characterised in that** the at least one resilient attaching element (54) takes the form of an annular body which is slit in the longitudinal direction.

4. Device according to claim 3, **characterised in that** the annular body has, in the region of the slit (55), sections (56A, 56B) which folded over in the outward direction on both sides.

5. Device according to one of claims 1 to 4, **characterised in that** the device for detecting moisture has a layer not permeable to liquid which is adjacent to the patient and situated on the outside and a layer (69) absorbent of liquid which is remote from the patient and is situated on the inside.

6. Device according to claims 5, **characterised in that** the layer (69) absorbent of liquid which is situated on the inside is a textile material which has the electrically conductive structure (72).

7. Device according to claim 5 or 6, **characterised in that** the layer (70) situated on the outside is a layer of resilient plastics material which has been thermally deformed.

8. Device according to claim 6 or 7, **characterised in that** the electrically conductive structure (72) has at least one electrical conductor (73, 74) which is embedded in the textile material (69).

9. Device according to claim 8, **characterised in that** the textile material is a woven material (69) having non-conductive warp filaments and non-conductive weft filaments (71) and conductive warp filaments and conductive weft filaments (71), which are so arranged that the at least one conductor (73, 74) is formed.

10. Device according to one of claims 1 to 9, **characterised in that** the device for detecting moisture has connecting contacts (66) to allow electrical contact to be made with the moisture sensor.

11. Device according to claim 10, **characterised in that** the connecting contacts (66) are formed on the end-piece (52A) of an elongated sub-section (79) of the device for detecting moisture.

12. Device according to one of claims 1 to 11, **characterised in that** the device for detecting moisture has two sub-sections or portions which are spaced apart from one another and which take the form of a first and a second resilient attaching element (59, 61).

13. Device according to claim 12, **characterised in that** the device for detecting moisture has a third resilient attaching element (54) which is arranged between the first and second resilient attaching elements (59,61).

14. Device according to claim 13, **characterised in that** the third resilient attaching element (54) is of a greater length in the longitudinal direction than the first and second attaching elements (59, 61).

15. Device for monitoring an access to a patient for a system by which, via a flexible line, a liquid is fed to a patient and/or a liquid is fed out from the patient, **characterised in that** the monitoring arrangement (B) has a device (50) for detecting moisture according to one of claims 1 to 14.

16. Method of producing a device for detecting moisture according to one of claims 1 to 14, having the following steps:
application of a textile material (69) having an electrical conductive structure to a thermally deformable substrate material (70),
cutting out from the textile material and the substrate material of a composite item having a preset outline,
thermal deformation of the composite item comprising the textile material and the substrate material which has been cut out, and
cooling of the thermally deformed composite item comprising the textile material and the substrate material.

## Revendications

1. Dispositif pour la détection de l'humidité pour un dispositif destiné à la surveillance d'un accès à un patient pour une installation avec laquelle, par le biais d'une conduite souple, un liquide est acheminé à un patient et/ou un liquide est évacué du patient, dans lequel le dispositif pour la détection de l'humidité présente un capteur d'humidité qui est conçu comme une structure électriquement conductrice (72),
**caractérisé en ce**
**que** le dispositif pour la détection de l'humidité est conçu comme un corps d'un seul tenant, élastique au moins par endroits, et qu'au moins une pièce partielle ou un segment du dispositif pour la détection de l'humidité est conçu(e) comme un élément de fixation élastique (54) avec une conduite souple ou un système de liaison des segments (54A, 54B) entourant la conduite souple.

2. Dispositif selon la revendication 1, **caractérisé en ce que** le dispositif pour la détection de l'humidité est conçu comme un corps allongé.

3. Dispositif selon la revendication 1 ou 2, **caractérisé en ce que** l'au moins un élément de fixation élastique (54) est conçu comme un corps annulaire qui est fendu en direction longitudinale.

4. Dispositif selon la revendication 3, **caractérisé en ce que** le corps annulaire présente dans la zone de la fente (55), des segments (56A, 56B) qui sont recourbés des deux côtés vers l'extérieur.

5. Dispositif selon l'une des revendications 1 à 4, **caractérisé en ce que** le dispositif pour la détection de l'humidité présente une couche extérieure (70) non perméable à l'humidité, tournée vers le patient, et une couche intérieure (69) absorbante, détournée du patient.

6. Dispositif selon la revendication 5, **caractérisé en ce que** la couche intérieure (69) absorbant le liquide est un matériau textile qui présente la structure électriquement conductrice (72).

7. Dispositif selon la revendication 5 ou 6, **caractérisé en ce que** la couche extérieure (70) est une couche composée d'une matière plastique élastique, qui est déformée thermiquement.

8. Dispositif selon la revendication 6 ou 7, **caractérisé en ce que** la structure électriquement conductrice (72) présente au moins une piste conductrice électrique (73, 74) qui est englobée dans le matériau textile (69).

9. Dispositif selon la revendication 8, **caractérisé en ce que** le matériau textile est un tissu (69) avec des fils de chaîne non conducteurs et des fils de trame non conducteurs (71) ainsi que des fils de chaîne conducteurs et des fils de trame conducteurs (71), qui sont agencés de telle sorte que l'au moins une piste conductrice électrique soit conçue.

10. Dispositif selon l'une des revendications 1 à 9, **caractérisé en ce que** le dispositif pour la détection de l'humidité présente des contacts de raccordement (66) pour la mise en contact électrique du capteur d'humidité.

11. Dispositif selon la revendication 10, **caractérisé en ce que** les contacts de raccordement (66) sur l'embout (52A) d'une pièce partielle allongée (79) du dispositif sont conçus pour la détection de l'humidité.

12. Dispositif selon l'une des revendications 1 à 11, **caractérisé en ce que** le dispositif pour la détection de l'humidité présente deux pièces partielles ou segments agencé(e)s à distance l'un(e) de l'autre, qui sont conçu(e)s comme un premier et un deuxième élément de fixation élastique (59, 61).

13. Dispositif selon la revendication 12, **caractérisé en ce que** le dispositif pour la détection de l'humidité présente un troisième élément de fixation élastique (54) qui est agencé entre le premier et le deuxième élément de fixation élastique (59, 61).

14. Dispositif selon la revendication 13, **caractérisé en ce que** le troisième élément de fixation élastique (54) a, en direction longitudinale, une longueur supérieure à celle du premier et du deuxième élément de fixation élastique (59, 61).

15. Dispositif destiné à la surveillance d'un accès à un patient pour une installation avec laquelle, par le biais d'une conduite souple, un liquide est acheminé à un patient et/ou un liquide est évacué du patient, **caractérisé en ce que** le dispositif de surveillance (B) présente un dispositif (50) pour la détection de l'humidité selon l'une des revendications 1 à 14.

16. Procédé de fabrication d'un dispositif pour la détection de l'humidité selon l'une des revendications 1 à 14, avec les étapes de procédé suivantes :
l'application d'un matériau textile (69) avec une structure électriquement conductrice sur un matériau de support thermiquement déformable (70),
la découpe d'un composite avec un contour prédéfini à partir du matériau textile et du matériau de support,
la déformation thermique du composite découpé à partir du matériau textile et du matériau de support, et
le refroidissement du composite déformé thermiquement à partir du matériau textile et du matériau de support.
